# EUROPEAN PATENT SPECIFICATION

(11) **EP 4 626 396 B1**
(45) Date of publication and mention of the grant of the patent: **20.05.2026**
(21) Application number: 23793723.0
(22) Date of filing: 18.10.2023
(51) Int. Cl.: A61K 8/72, A61K 8/44, A61K 8/9728, A61Q 19/00, A61K 8/34, A61K 8/73, A61K 8/19, A61K 8/49, A61K 8/40, A61K 8/35, A61K 8/67, A61K 8/31, A61K 8/37, A61K 8/81, A61K 8/891, A61K 8/365

(54) **A PERSONAL CARE COMPOSITION**
KÖRPERPFLEGEZUSAMMENSETZUNG
COMPOSITION DE SOINS PERSONNELS

(30) Priority: 28.11.2022 WO PCT/CN2022/134601; 12.01.2023 EP 23151221
(43) Date of publication of application: 08.10.2025
(73) Proprietor: Unilever IP Holdings B.V., 6708 WH Wageningen (NL); Unilever Global IP Limited, Wirral, Merseyside CH62 4ZD (GB)
(72) Inventor: CHEN, Guoqiang, 6708 WH Wageningen (NL); WANG, Xiaoli, 6708 WH Wageningen (NL); WANG, Yun, 6708 WH Wageningen (NL); YANG, Xiaoxia, 6708 WH Wageningen (NL)
(74) Representative: Unilever Patent Group
(86) International application number: PCT/EP2023/078916
(87) International publication number: WO 2024/115001

(56) References cited:
- CN-A- 108 743 450
- CN-A- 113 398 020
- US-A1- 2017 326 062
- ANONYMOUS: "Ingredient Trends 2020-2021, Part 2 | Global Cosmetic Industry", 27 October 2020 (2020-10-27), XP055883421, Retrieved from the Internet <URL:https://www.gcimagazine.com/ingredients/regulatory/article/21849141/ingredient-trends-2020-2021-part-2> [retrieved on 20220125]
- ANONYMOUS: "GRANPOWDER BBP-700 100% NATURALLY DERIVED, BIO-BASED POWDER", 14 July 2021 (2021-07-14), pages 1 - 2, XP093041148, Retrieved from the Internet <URL:https://web.archive.org/web/20210714084554if_/https://digital.h5mag.com/digital/edit-beauty_horizons_3_2021_ita/company_insight_imcd/150223/Texturizzante___GRANPOWDER_BBP_700.pdf> [retrieved on 20230421]
- GRECO LETTERIA ET AL: "Evaluation of the Soothing and Protective Properties of a Lignin Hydrolyzate", COSMETICS, vol. 6, no. 3, 3 July 2019 (2019-07-03), pages 38, XP093055472, DOI: 10.3390/cosmetics6030038

## Description

### Field of the Invention

The present invention relates to a personal care composition. In particular, the present invention is related to a personal care composition, as defined in the appended claims, comprising ferment powder and lignin compound for blocking pollutant.

### Background of the Invention

Air pollution is a big problem, particularly in some of the developing countries. Particulate matter (PM) is one of the important to affect the air quality which are inhalable particles composed of sulphate, nitrates, ammonia, sodium chloride, black carbon, mineral dust and water. Particles with a diameter of less than 10 microns (PM10), including fine particles less than 2.5 microns (PM2.5) pose the greatest risks to health, as they can enter the lungs and the bloodstream. In addition, PM may bring adverse effects of pollution on human skin. These adverse effects include premature ageing, development of fine lines and wrinkles, pigmented spots, hyperpigmentation, rash and inflammation. See in this context: "GRECO LETTERIA ET AL: "Evaluation of the Soothing and Protective Properties of a Lignin Hydrolyzate",COSMETICS, vol. 6, no. 3, 3 July 2019 (2019-07-03), page 38, XP093055472,DOI: 10.3390/cosmetics6030038".

Therefore, we have recognized there is a need to develop a person care composition which is able to protect the skin from harmful effects of atmospheric pollutants. The present inventors developed a personal care composition comprising ferment powder and lignin compound, wherein the ferment powder has an average diameter of 0.1 to 100 microns. It was surprisingly found that such composition is capable of synergistically boosting the pollutant blocking efficacy.

### Summary of the Invention

The invention is defined in the claims.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

Where a feature is disclosed with respect to a particular aspect of the invention (for example a composition of the invention), such disclosure is also to be considered to apply to any other aspect of the invention (for example a method of the invention) mutatis mutandis.

"Diameter" as used herein refers to particle diameter in non-aggregated state unless otherwise stated. For polydisperse samples having particulate with diameter no greater than 1 µm, diameter means the z-average diameter measured, for example, using dynamic light scattering (see international standard ISO 13321) with an instrument such as a Zetasizer Nano^{™} (Malvern Instruments Ltd, UK) unless otherwise stated. For polydisperse samples having particulate with diameter no less than 1 µm, diameter means the apparent volume median diameter (D50, also known as x50 or sometimes d(0.5)) of the particles measurable for example, by laser diffraction using a system (such as a Mastersizer^{™} 2000 available from Malvern Instruments Ltd) meeting the requirements set out in ISO 13320 unless otherwise stated.

"Ferment" as used herein refers to ingredient obtained from microorganisms by a fermentation process. Fermentation process means the metabolic process that produces chemical changes in organic substrates through the action of enzymes and the fermentation process occurs by the controlled use of microorganisms. In the absence of oxygen, the fermentation process typically converts sugars, such as, glucose, lactose, whey or propionic acids into polyesters. Preferably, the ferment is polyester obtained by fermentation process. Such polyester is biodegradable, whereby microorganisms are able to enzymatically metabolize the polyester through either an anaerobic or aerobic biochemical process.

Preferably, the microorganism for providing the ferment is yeast or bacteria, more preferably yeast, even more preferably the ferment is *Saccharomyces.* The suitable bacteria may be selected from *Cupriavidus necator, Alcaligenes latus, Alcaligenes eutrophus, Escherichia coli, Pseudomonas putida, and Aeromona hydrophila.*

Preferably, the ferment is yeast ferment, more preferably *Saccharomyces* ferment. Yeast (*Saccharomyces*) ferment refers to the ferment obtained from yeast (*Saccharomyces*) via a fermentation process.

The ferment comprises polyhydroxyalkanoate (PHA). More preferably, the PHA is polyhydroxybutyrate (PHB), polyhydroxyvalerate (PHV), polyhydroxybutyratevalerate (PHBV), or polyhydroxyhexanoate (PHH), or a combination thereof. Even more preferably the ferment comprises polyhydroxybutyrate. In certain embodiments, the polyesters or ferments are those which comprise at least one of the following polymeric and/or copolymeric structures: poly-3-hydroxybutyrate (P-3HB), poly-3-hydroxy-butyrate-co-3-hydroxyvalerate (P-3HB-3HV), poly-3-hydroxybutyrate-co-4-hydroxybutyrate (P-3HB-4HB), and poly-3-hydroxybutyrate-co-3-hydroxyvalerate-co-4-hydroxybutyrate (P-3HB-3HV-4HB).

To improve the sensory, and/or tactile feeling, the ferment powder preferably comprises the ferment and amino acid derivative. The amino acid derivative as used in the present invention typically refers to ester and/or amide of the amino acid. Preferably, the amino acid derivative is N-Acylated amino acid. More preferably, the amino acid derivative is selected from N-capryloyl amino acid, N-undecylenoyl amino acid, N-palmitoyl-amino acid, N-cocoyl amino acid, N-acetyl amino acids, N-oleoyl amino acid, N-lauroyl amino acid, and N-stearoyl amino acid. Even more preferably, the amino acid derivative is selected from N6-lauroyl-L-lysine (lauroyl lysine), N6-capryloyl-L-lysine, undecylenoyl phenylalanine, undecylenoyl glycine, palmitoyl arginine, palmitoyl glycine, palmitoyl proline, palmitoyl serine, palmitoyl lysyl aminovaleroyl lysine, N-cocoyl glutamic acid, capryloyl glycine, capryloyl serine, acetyl cysteine, acetyl glutamic acid, acetyl glutamine, diethyl acetyl aspartate, oleoyl tyrosine, lauroyl aspartate, and stearoyl glutamic acid. Preferably, the amino acid derivative is lysine derivative, more preferably N-acylated lysine, even more preferably N6-lauroyl-L-lysine.

Preferably, the ferment powder comprises 55 to 98% of ferment by weight of the powder and 2 to 45% of amino acid derivative by weight of the powder. Preferably the amount of ferment is from 80 to 95% by weight of the powder and the amount of amino acid derivative is from 5 to 20% by weight of the powder. Preferably the ferment powder comprises ferment and amino acid derivative. More preferably, the ferment powder comprises ferment and N-acylated amino acid. Even more preferably, the ferment powder comprises yeast ferment and N-acylated amino acid. Still even more preferably, the ferment powder has an INCI (International Nomenclature of Cosmetic Ingredient) name of Saccharomyces Ferment (and) Lauroyl Lysine.

The ferment powder has an average diameter of 0.2 to 60 microns, even more preferably 0.5 to 40 microns, still even more preferably 1 to 20 microns and most preferably 2 to 10 microns. Preferably, the ferment powder has spherical shape.

The ferment powder has the capability of absorbing oils. Preferably, the ferment powder has an oil absorption value of 30g/100g to 1000g/100g, more preferably 50g/100g to 600g/100g, even more preferably 100g/100g to 400g/100g and most preferably 150g/100g to 300g/100g. The oil absorption value refers to the values measured in conformity with ASTM Method D281-84.

When preparing the ferment powder, the ferment and the amino acid derivative are thoroughly intermingled with one another, forming a substantially uniform mixture and, after micronization to form the ferment powder. The ferment powders are preferably free-flowing powders. Preferably, the ferment powders have a bulk density of from 0.1 g/mL to 0.4 g/mL, more preferably 0.15 g/mL to 0.3 g/mL and even more preferably 0.18 to 0.25 g/mL.

Preferably, the ferment powder is present in amount of 0.01 to 15% by weight of the composition, more preferably 0.1 to 12%, even more preferably 0.5 to 9%, still even more preferably 1 to 7% and most preferably 2 to 4% by weight of the composition.

"Lignin compound" as used herein refers to a compound comprising lignin or a modified lignin. Typically the modified lignin means lignin which has been modified through alkylation, alkoxylation, sulfonation, sulfation, alkoxy sulfation, sulfomethylation or combinations thereof.

The lignin compound comprises lignin, lignosulfonate, cationically-modified lignin, amino lignin, alkylated lignin, crosslinked lignosulfonate, crosslinked lignin or a combination thereof. More preferably the lignin compound comprises lignin, lignosulfonate, or a combination thereof. Even more preferably the lignin compound comprises lignosulfonate. Still even more preferably the lignin compound is lignosulfonate.

Lignosulphonate refers to sulphonated lignin. The lignosulphonate may be provided in the form of a salt with a suitable cation, for example sodium, magnesium, ammonium, calcium or a combination thereof. More preferably the lignosulphonate is sodium lignosulphonate, magnesium lignosulphonate or a mixture thereof and most preferably the lignosulphonate is sodium lignosulphonate. Sodium lignosulphonate is exchangeable with sodium lignin sulfonate. Preferably the lignin compound comprises sodium lignosulfonate and most preferably the lignin compound is sodium lignosulfonate.

Preferably, the lignin compound has a weight-average molecular weight of from 300 to 1,000,000, and more preferably from 500 to 500,000. Preferably, the lignosulphonate has a weight-average molecular weight of from 300 to 1,000,000, and more preferably from 500 to 500,000.

Preferably, the lignin compound is present in amount of 0.01 to 20% by weight of the composition, more preferably 0.1 to 18%, even more preferably 0.5 to 15%, still even more preferably 1 to 10% and most preferably 2 to 8% by weight of the composition. Preferably, the lignosulfonate is present in amount of 0.01 to 20% by weight of the composition, more preferably 0.1 to 18%, even more preferably 0.5 to 15%, still even more preferably 1 to 10% and most preferably 2 to 8% by weight of the composition.

Preferably, the weight ratio of the lignin compound to the ferment powder is 1:20 to 70:1, more preferably 1:8 to 25:1, even more preferably 1:3 to 9:1, and still even more preferably 1:1 to 3:1. Preferably, the weight ratio of the lignosulphonate to the ferment powder is 1:20 to 70:1, more preferably 1:8 to 25:1, even more preferably 1:3 to 9:1, and still even more preferably 1:1 to 3:1.

The composition may comprise water in amount of 35 to 95% by weight of the composition, more preferably from 45 to 92%, even more preferably from 50 to 90%, most preferably from 68% to 88% by weight of the composition. The composition is preferably an emulsion and more preferably an oil-in-water emulsion.

The composition may comprise resorcinol derivative. Resorcinol derivative preferably refers to that at least one hydrogen on the ring structure and/or on a hydroxy group of the resorcinol replaced with an alkyl group, phenyl alkyl group. Preferably, the resorcinol derivative is 4-substituted resorcinol. Preferably, the resorcinol derivative is selected from 4-ethyl resorcinol, 4-butyl resorcinol, 4-hexyl resorcinol, phenylethyl resorcinol, or a mixture thereof, and more preferably, the resorcinol derivative comprises 4-hexyl resorcinol. The amount of the resorcinol derivative is preferably in the range of 0.00001 to 10%, more preferably from 0.001 to 5% and most preferably from 0.1 to 0.6% by weight of the total amount of the composition.

Preferably, the composition comprises Vitamin B3 compounds (including derivatives of vitamin B3). The vitamin B3 compounds comprises niacin, nicotinic acid, niacinamide or a mixture thereof. The most preferred vitamin B3 compound is niacinamide. Amount of Vitamin B3 compounds may be 0.1 to 10%, preferably 0.5 to 5% by weight of the composition.

The composition preferably additionally comprises one or more organic sunscreens. A wide variety of organic sunscreen is suitable for use in combination with the essential ingredients of this invention. Suitable UV-A / UV-B sunscreen include, 2- hydroxy-4-methoxybenzophenone, octyldimethyl p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethyl- aminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonicbenzoxazoic acid, 2-ethylhexyl-p-methoxycinnamate, butylmethoxydibenzoylmethane, 2-hydroxy-4- methoxybenzophenone, octyldimethyl-p-aminobenzoic acid and mixtures thereof. The most suitable organic sunscreens are 2-ethylhexyl-p-methoxycinnamate, butylmethoxydibenzoylmethane or a mixture thereof. A safe and effective amount of organic sunscreen may be used in the compositions useful in the subject invention. The composition preferably comprises from 0.1% to 10%, more preferably from 0.1% to 5%, of organic sunscreen by weight of the composition.

Preferably, the composition comprises polyhydric alcohol. Polyhydric alcohols may be selected from group of glycerin, propylyene glycol, dipropylene glycol, polypropylene glycol, polyethylene glycol, sorbitol, hydroxypropyl sorbitol, hexylene glycol, 1,3-butylene glycol, isoprene glycol, ethoxylated glycerol, propoxylated glycerol or a mixture thereof. Most preferred polyhydric alcohol is glycerol known also as glycerin. The amount of polyhydric alcohol may range anywhere from 0.1 to 20%, preferably 0.5 to 15% and more preferably 2 and 10% by weight of the composition.

Water-insoluble skin benefit agents may also be formulated into the compositions as conditioners and moisturizers. Examples include silicone oils; and vegetable triglycerides such as sunflower seed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

Preferably, the composition has a viscosity of at least 10 mPa·s, more preferably in the range 30 to 10000 mPa·s, even more preferably 50 to 5000 mPa·s, and most preferably 100 to 2000 mPa·s, when measured at 20 degrees C at a relatively high shear rate of about 20 s⁻¹. Preferably, the composition is in the form of fluid at 25 degrees C and atmospheric pressure.

Preferably, the personal care composition is a skin care composition. The term "skin" as used herein includes the skin on the face, neck, chest, abdomen, back, arms, under arms, hands, and legs. Preferably "skin" means includes the skin on the face and under arms, more preferably skin means skin on the face other than lips and eyelids.

Preferably, preventing pollutant refers to reducing contact of skin with a pollutant. More preferably, preventing pollutant comprising reducing deposition of pollutant on composition applied onto the surface and/or blocking pollutant in the composition applied onto the surface. Preferably, pollutant is particulate matter, more preferably PM2.5 and/or PM10.

The following examples are provided to facilitate an understanding of the invention. The examples are not intended to limit the scope of the claims.

### Examples

### Materials

| Supplier | Trade name | INCI name | Average diameter (µm) | Oil absorption (9/1009) |
|---|---|---|---|---|
| Tokyo Chemical Industry Co. Ltd | - | Sodium lignin sulfonate | - | - |
| Grant Industries, Inc. | Granpowder BBP-700 | Saccharomyces Ferment (and) Lauroyl Lysine | 2-10 | 200-250 |

### Example 1

This example demonstrates the synergistic effect for preventing pollutant deposition by combing sodium lignin sulfonate and saccharomyces ferment.

**Table 1**

| Ingredient | Sample (wt%)* | | |
|---|---|---|---|
| | A | B | 1 |
| Water | To 100 | To 100 | |
| Granpowder BBP-700 | 3.00 | - | 3.00 |
| Sodium Lignin Sulfonate | - | 5.00 | 5.00 |
| Glycerin | 2.50 | 2.50 | 2.50 |
| Disodium EDTA | 0.10 | 0.10 | 0.10 |
| Carbopol Ultrez 10 | 0.40 | 0.40 | 0.40 |
| KOH | 0.54 | 0.54 | 0.54 |
| Xanthan Gum | 0.30 | 0.30 | 0.30 |
| NaOH | 0.22 | 0.22 | 0.22 |
| Phenylbenzimidazole Sulfonic Acid | 1.50 | 1.50 | 1.50 |
| Parsol 1789 | 1.00 | 1.00 | 1.00 |
| Octocrylene | 1.00 | 1.00 | 1.00 |
| Ethylhexyl Salicylate | 4.00 | 4.00 | 4.00 |
| Dimethicone 50 cSt | 1.00 | 1.00 | 1.00 |
| Glycol Stearate & Stearamide AMP | 1.39 | 1.39 | 1.39 |
| PEG-100 Stearate | 1.19 | 1.19 | 1.19 |
| Cetyl Alcohol | 0.37 | 0.37 | 0.37 |
| Glyceryl Stearate | 0.65 | 0.65 | 0.65 |
| Stearic Acid | 2.35 | 2.35 | 2.35 |
| BHT | 0.05 | 0.05 | 0.05 |
| Petrolatum | 0.01 | 0.01 | 0.01 |
| Phenoxyethanol | 0.40 | 0.40 | 0.40 |
| Niacinamide | 0.10 | 0.10 | 0.10 |
| Caprylyl Glycol | 0.50 | 0.50 | 0.50 |

| | | | |
|---|---|---|---|
| *The level in this table refers the level of left-listed ingredients, unless otherwise specified. | | | |

The personal care compositions in Table 1 were formulated by following standard procedure.

The prepared samples A, B and 1 were applied onto six identical glass plates (22 mm × 22 mm) evenly with a same amount and dried overnight to form same dry cream films. Then coated glass plates were placed into a home-made pollution simulation chamber with a temperature of 25 °C and relative humidity of 50%. Vehicle exhausts, as simulation of particulate matters, were supplied into the closed chamber at level of 400 µg/m³ for 8 hours. Then, the coated glass plates were kept in the closed chamber overnight.

Then, the coated samples (a thin film on glass plate) with deposited pollution were peeled off by new tapes for five (5) times. Each tape was washed by acetone and the amounts of PAH and octocrylene (as marker of depth) on each tape were determined by GC-MS (Thermo scientific ISQ 7000 Single Quadrupole Mass Spectrometer and Thermo scientific Trace 1300 Gas Chromatograph).

The amount of PAH (Polycyclic Aromatic Hydrocarbons) on coated glass plates were determined by GC-MS (Thermo scientific ISQ 7000 Single Quadrupole Mass Spectrometer and Thermo scientific Trace 1300 Gas Chromatograph) and summarized in Table 2.

Under the assumption that the concentration of octocrylene is consistent along the height of the film, the amount of octocrylene represents the height of the film formed by samples. A fitted equation for each sample was generated by plotting the data of accumulative PAH percentage and the height of the film. Then, the percentage of PAH blocked at the top 20% height of coated sample film based on the total PAH blocked by the sample film for each sample was obtained from the fitted equation. The results were summarized in Table 2.

**Table 2**

| Sample | Granpowder BBP-700 | Sodium Lignin Sulfonate | Blocked PAH at top 20% height (%) |
|---|---|---|---|
| A | 3.00 | - | 25 |
| B | - | 5.00 | 31 |
| 1 | 3.00 | 5.00 | 100 ^{#} |

| | | | |
|---|---|---|---|
| ^{#}: Significantly better than A, B or A+B. | | | |

As evidenced in Table 2, the skin care composition containing saccharomyces ferment and sodium lignin sulfonate (Sample 1) provided a significantly better PAH blocking efficacy at the top 20% height of coated sample film of the skin care composition than skin care composition containing saccharomyces ferment only plus skin care composition containing sodium lignin sulfonate only (Sample A and B). It was surprisingly found that the pollutant blocking efficacy was synergistically boosted by combining saccharomyces ferment and sodium lignin sulfonate.

## Claims

1. A personal care composition comprising:
(a) ferment powder; and
(b) lignin compound,
wherein
(i) the ferment powder has an average diameter of 0.2 to 60 microns;
(ii) the ferment comprises polyhydroxyalkanoate; and
(iii) the lignin compound comprises lignin, lignosulfonate, cationically modified lignin, amino lignin, alkylated lignin, crosslinked lignosulfonate, crosslinked lignin or a combination thereof.

2. The composition according to claim 1 wherein the lignin compound comprises lignosulfonate.

3. The composition according to claim 2 wherein the lignin compound is present in amount of 0.01 to 20% preferably 0.5 to 15% by weight of the composition.

4. The composition according to any one of the preceding claims wherein the ferment is yeast ferment, preferably *Saccharomyces* ferment.

5. The composition according to any one of the preceding claims wherein the polyhydroxyalkanoate is polyhydroxybutyrate (PHB), polyhydroxyvalerate (PHV), polyhydroxybutyratevalerate (PHBV), or polyhydroxyhexanoate (PHH).

6. The composition according to any one of the preceding claims wherein the ferment powder preferably comprises ferment and amino acid derivative, preferably the ferment powder preferably comprises ferment and N-Acylated amino acid, and more preferably the ferment powder has an INCI name of Saccharomyces Ferment (and) Lauroyl Lysine.

7. The composition according to any one of the preceding claims wherein the ferment powder has an average diameter of 1 to 20 microns and preferably 2 to 10 microns.

8. The composition according to any one of the preceding claims wherein the ferment powder has an oil absorption value of 30g/100g to 1000g/100g, preferably 100g/100g to 400g/100g.

9. The composition according to any one of the preceding claims wherein the ferment powders have a bulk density of from 0.1 g/mL to 0.4 g/mL, preferably 0.18 to 0.25 g/mL.

10. The composition according to any one of the preceding claims wherein the ferment powder is present in amount of 0.01 to 15% preferably 1 to 7% by weight of the composition.

11. The composition according to any one of the preceding claims wherein the weight ratio of the lignin compound to the ferment powder is 1:8 to 25:1, preferably 1:1 to 3:1.

12. The composition according to any one of the preceding claims wherein the composition comprises water in amount of 45 to 92% by weight of the composition.

13. The composition according to any one of the preceding claims wherein the composition is an oil-in-water emulsion.

14. Use of a composition according to any one of the preceding claims for preventing pollutant from skin, in particular PM2.5 and/or PM10 particulate pollutant.

15. A method of preventing pollutant from skin, comprising a step of applying the composition according to any one of claims 1 to 13 to the skin.

## Patentansprüche

1. Körperpflegezusammensetzung, umfassend:
(a) Fermentpulver; und
(b) eine Ligninverbindung, wobei
(i) das Fermentpulver einen durchschnittlichen Durchmesser von 0,2 bis 60 Mikrometern aufweist;
(ii) das Ferment Polyhydroxyalkanoat umfasst; und
(iii) die Ligninverbindung Lignin, Lignosulfonat, kationisch modifiziertes Lignin, Aminolignin, alkyliertes Lignin, vernetztes Lignosulfonat, vernetztes Lignin oder eine Kombination davon umfasst.

2. Zusammensetzung nach Anspruch 1, wobei die Ligninverbindung Lignosulfonat umfasst.

3. Zusammensetzung nach Anspruch 2, wobei die Ligninverbindung in einer Menge von 0,01 bis 20 Gew.-% der Zusammensetzung vorhanden ist, vorzugsweise von 0,5 bis 15 Gew.-%.

4. Zusammensetzung nach einem der vorhergehenden Ansprüche, wobei das Ferment ein Hefeferment, vorzugsweise ein *Saccharomyces-Ferment,* ist.

5. Zusammensetzung nach einem der vorhergehenden Ansprüche, wobei das Polyhydroxyalkanoat Polyhydroxybutyrat (PHB), Polyhydroxyvalerat (PHV), Polyhydroxybutyratvalerat (PHBV) oder Polyhydroxyhexanoat (PHH) ist.

6. Zusammensetzung nach einem der vorhergehenden Ansprüche, wobei das Fermentpulver vorzugsweise Ferment und Aminosäurederivat umfasst, wobei das Fermentpulver vorzugsweise Ferment und N-acylierte Aminosäure umfasst und das Fermentpulver bevorzugter einen INCI-Namen vom Saccharomyces-Ferment (und) Lauroyl-Lysin aufweist.

7. Zusammensetzung nach einem der vorhergehenden Ansprüche, wobei das Fermentpulver einen durchschnittlichen Durchmesser von 1 bis 20 Mikrometern und vorzugsweise von 2 bis 10 Mikrometern aufweist.

8. Zusammensetzung nach einem der vorhergehenden Ansprüche, wobei das Fermentpulver einen Ölabsorptionswert von 30 g/100 g bis 1000 g/100 g, vorzugsweise von 100 g/100 g bis 400 g/100 g, aufweist.

9. Zusammensetzung nach einem der vorhergehenden Ansprüche, wobei das Fermentpulver eine Schüttdichte von 0,1 g/ml bis 0,4 g/ml, vorzugsweise 0,18 bis 0,25 g/ml, aufweist.

10. Zusammensetzung nach einem der vorhergehenden Ansprüche, wobei das Fermentpulver in einer Menge von 0,01 bis 15 Gew.-% der Zusammensetzung vorliegt, vorzugsweise von 1 bis 7 Gew.-%.

11. Zusammensetzung nach einem der vorhergehenden Ansprüche, wobei das Gewichtsverhältnis der Ligninverbindung zum Fermentpulver 1:8 bis 25:1, vorzugsweise 1:1 bis 3:1, beträgt.

12. Zusammensetzung nach einem der vorhergehenden Ansprüche, wobei die Zusammensetzung Wasser in einer Menge von 45 bis 92 Gew.-% der Zusammensetzung umfasst.

13. Zusammensetzung nach einem der vorhergehenden Ansprüche, wobei die Zusammensetzung eine Öl-in-Wasser-Emulsion ist.

14. Verwendung einer Zusammensetzung nach irgendeinem der vorhergehenden Ansprüche zum Verhindern von Hautverschmutzung, insbesondere durch partikelförmige Schmutzstoffe PM2,5- und/oder PM10.

15. Verfahren zum Verhindern von Hautverschmutzung, umfassend einen Schritt des Aufbringens der Zusammensetzung nach irgendeinem der Ansprüche 1 bis 13 auf die Haut.

## Revendications

1. Composition de soins personnels comprenant :
(a) une poudre de ferment ; et
(b) un composé de lignine,
dans laquelle
(i) la poudre de ferment a un diamètre moyen de 0,2 à 60 micromètres ;
(ii) le ferment comprend un polyhydroxyalcanoate ; et
(iii) le composé de lignine comprend la lignine, un lignosulfonate, une lignine cationiquement modifiée, une aminolignine, une lignine alkylée, un lignosulfonate réticulé, une lignine réticulée ou une de leurs combinaisons.

2. Composition selon la revendication 1, dans laquelle le composé de lignine comprend un lignosulfonate.

3. Composition selon la revendication 2, dans laquelle le composé de lignine est présent en une quantité de 0,01 à 20 %, de préférence de 0,5 à 15 % en poids de la composition.

4. Composition selon l'une quelconque des revendications précédentes, dans laquelle le ferment est un ferment de levure, de préférence un ferment de *Saccharomyces.*

5. Composition selon l'une quelconque des revendications précédentes, dans laquelle le polyhydroxyalcanoate est un polyhydroxybutyrate (PHB), un polyhydroxyvalérate (PHV), un polyhydroxybutyrate-valérate (PHBV), ou un polyhydroxyhexanoate (PHH).

6. Composition selon l'une quelconque des revendications précédentes, dans laquelle la poudre de ferment comprend de préférence un ferment et un dérivé d'acide aminé, de préférence la poudre de ferment comprend de préférence un ferment et un acide aminé N-acylé, et mieux encore la poudre de ferment a un nom INCI de ferment de Saccharomyces (et) lauroyl-lysine.

7. Composition selon l'une quelconque des revendications précédentes, dans laquelle la poudre de ferment a un diamètre moyen de 1 à 20 micromètres et de préférence de 2 à 10 micromètres.

8. Composition selon l'une quelconque des revendications précédentes, dans laquelle la poudre de ferment a une valeur d'absorption d'huile de 30 g/100 g à 1000 g/100 g, de préférence de 100 g/100 g à 400 g/100 g.

9. Composition selon l'une quelconque des revendications précédentes, dans laquelle les poudres de ferment ont une masse volumique apparente de 0,1 g/ml à 0,4 g/ml, de préférence de 0,18 à 0,25 g/ml.

10. Composition selon l'une quelconque des revendications précédentes, dans laquelle la poudre de ferment est présente en une quantité de 0,01 à 15 %, de préférence de 1 à 7 % en poids de la composition.

11. Composition selon l'une quelconque des revendications précédentes, dans laquelle le rapport en poids du composé de lignine à la poudre de ferment est de 1/8 à 25/1, de préférence de 1/1 à 3/1.

12. Composition selon l'une quelconque des revendications précédentes, dans laquelle la composition comprend de l'eau en une quantité de 45 à 92 % en poids de la composition.

13. Composition selon l'une quelconque des revendications précédentes, dans laquelle la composition est une émulsion huile dans l'eau.

14. Utilisation d'une composition selon l'une quelconque des revendications précédentes pour protéger la peau contre un polluant, en particulier un polluant particulaire PM2.5 et/ou PM10.

15. Méthode de protection de la peau contre un polluant, comprenant une étape d'application sur la peau de la composition selon l'une quelconque des revendications 1 à 13.
